(19) Europäisches Patentamt
European Patent Office
Office européen des brevets

(11) Veröffentlichungsnummer: **0 146 106**
**A2**

# (12) EUROPÄISCHE PATENTANMELDUNG

(21) Anmeldenummer: **84115120.2**

(22) Anmeldetag: **10.12.84**

(51) Int. Cl.⁴: **A 61 K 7/46**, A 61 L 9/04

(30) Priorität: **19.12.83 DE 3345847**

(43) Veröffentlichungstag der Anmeldung: **26.06.85**
**Patentblatt 85/26**

(84) Benannte Vertragsstaaten: **AT BE CH DE FR GB IT LI NL SE**

(71) Anmelder: **Henkel Kommanditgesellschaft auf Aktien,**
**Postfach 1100 Henkelstrasse 67,**
**D-4000 Düsseldorf-Holthausen (DE)**

(72) Erfinder: **Dohr, Manfred, Dr., Im Diepental 13,**
**D-4000 Dsseldorf 13 (DE)**
Erfinder: **Wiemers, Norbert, Dr.,**
**Robert-Koch-Strasse 17, D-4019 Monheim (DE)**
Erfinder: **Gruber, Werner, Dr., Franz-Kremer-Strasse 7,**
**D-4052 Korschenbroich 1 (DE)**

## (54) Thermoplastische Polyamide als Duftstoffträger.

(57) Verwendung von an sich bekannten thermoplastischen Polyamiden auf Basis von dimerisierten Fettsäuren und vorwiegend aliphatischen Diaminen, insbesondere aber Polyetherdiaminen, als Träger für Duft- und Riechstoffe.

EP 0 146 106 A2

0146106

HENKEL KGaA
ZR-FE/Patente

Henkelstraße 67
4000 Düsseldorf, den 26. Juli 1984
Dr.SchOe/Br/Ge

# P a t e n t a n m e l d u n g

D 6825 EP

## "Thermoplastische Polyamide als Duftstoffträger"

Die vorliegende Erfindung betrifft die Verwendung von bestimmten thermoplastischen Polyamiden als Duft- oder Riechstoffträger.

Es ist bekannt, sowohl wasser- bzw. wasseralkohollösliche Kunststoffe als Träger für Riechstoffe einzusetzen. Unter den wasserlöslichen sind insbesondere die hydrophilen, wasserlöslichen Polyacrylsäureester zu erwähnen sowie quaternisiertes Polyvinylpyrrolidon (US-PS 36 81 248 und US-PS 39 39 099).

Unter den wasserunlöslichen ist insbesondere das Polyethylen und das Polypropylen zu erwähnen. In manchen Fällen verwendet man auch Kombinationen von Polyolefinen wie insbesondere Polyethylen hoher Dichte und niedriger Dichte (vgl. US-PS 35 67 119, US-PS 37 75 227, CA-PS 844 014). Ein weiterer, häufig für diesen Zweck verwendeter Kunststoff ist das PVC oder aber auch Polyurethanschaumstoff (vgl. Franz. PS 16 02 575). Weiterhin hat man als Duft- oder Riechstoffträger Polyvinylacetat oder thermoplastische Polyester oder Polyamide auf Basis von Dicarbonsäuren und Diaminen für diesen Zweck eingesetzt.

Die genannten Polymerverbindungen, die gewünschtenfalls auch mit Weichmachern, Harzen oder Füllstoffen verwendet werden können, befriedigen aber in mancher Hinsicht wie beispielsweise einer lang anhaltenden Freigabe des Duftstoffs nicht.

Aufgabe der vorliegenden Erfindung war es, solche Träger für Duft- bzw. Riechstoffe zu finden, die einerseits ein gutes Rückhaltevermögen für diese Verbindungen haben, andererseits aber auch ziemlich gleichmäßig den Wirkstoff an die Umgebung abgeben. Zwar war es bekannt, daß man in vielen Fällen bei handelsüblichen thermoplastischen Polyamiden ein gutes Release-Verhalten antrifft, jedoch lassen die bisher für diesen Zweck empfohlenen Polyamide noch bezüglich der Gleichmäßigkeit der Duftstoffabgabe an die Umgebung zu wünschen übrig.

Es wurde nun gefunden, daß Polyamide, die aus Dimerfettsäuren und Diaminen hergestellt sind, wesentlich günstiger hinsichtlich ihres Release-Verhaltens zu beurteilen sind, als die üblichen Polykondensate aus Dicarbonsäuren und Diaminen.

Die Erfindung betrifft somit die Verwendung von an sich bekannten thermoplastischen Polyamiden auf Basis von dimerisierten Fettsäuren und vorwiegend aliphatischen Diaminen als Träger für Duft- und Riechstoffe. Insbesondere aber betrifft sie die Verwendung von thermoplastischen Polyamiden, die als aliphatische Diamine zu einem erheblichen Teil Polyetherdiamine enthalten.

Diese thermoplastischen Polyamide sind seit langer Zeit bekannt. Beispielsweise kann man geeignete Polyamide aus den sogenannten Dimerfettsäuren, die durch "Polymerisation" ungesättigter Fettsäuren erhalten werden und aliphatischen und/oder cycloaliphatischen und/oder aromatischen und/oder heterocyclischen und/oder arylaliphatischen Diamen herstellen. Geeignete Diamine sind beispielsweise Ethylendiamin, Hexamethylendiamin oder auch Piperazin oder Diaminodiphenylmethan sowie oligomere endständige Aminogruppen enthaltende Polyether

...

(vgl. US-PS 42 18 351). Dabei soll der Anteil an kurzkettigen Komponenten oberhalb etwa 60 Mol-% liegen und der Anteil, der das Gerüst der dimerisierten Fettsäuren enthält, unterhalb von etwa 40 Mol-%. Weiterhin sind thermoplastische hinsichtlich der erwähnten Komponenten ähnlich aufgebaute Polyamide aus der GB-PS 13 19 807 bekannt, sie unterscheiden sich nur etwas bezüglich der Ethergruppen enthaltenden Aminkomponente. So sind denn die von Polyethergruppen enthaltenden Diamine (Polyetherdiamine) sich ableitenden Polyamide denen, die sich von kurzkettigen aliphatischen Diaminen - insbesondere Ethylendiamin - ableitenden Polyamiden in Bezug auf das Release-Verhalten noch überlegen.
Nach einer besonders bevorzugten Ausführungsform betrifft die Erfindung die Verwendung von thermoplastischen Polyamiden als Träger für Duft- und Riechstoffe, die durch Kondensation erhalten wurden von

a) 40 bis 48 Mol-% dimeren Fettsäuren,
b) 2 bis 10 Mol-% monomeren Fettsäuren,
c) 4 bis 25 Mol-% Polyetherdiaminen und
d) 25 bis 46 Mol-% aliphatischen Diamen.

Als Bausteine für die Polyamine sind unter den Polyetherdiaminen insbesondere solche geeignet, die der allgemeinen Formel

$$H_2N - R_1 - O - (R\ O)_x - R_2 - NH_2,$$

in der X eine Zahl zwischen 8 und 80, vornehmlich zwischen 8 und 40 und $R_1$ und $R_2$ gleiche oder verschiedene aliphatische und/oder cycloaliphatische Kohlenwasserstoffreste und R ein gegebenenfalls verzweigter aliphatischer Kohlenwasserstoffrest mit 1 bis 6 Kohlenstoff-

...

atomen darstellt, entsprechen.

Die bei der Polyamidherstellung einzusetzenden dimerisierten Fettsäuren werden häufig als polymere Fettsäuren bezeichnet und durch Polymerisation ungesättigter Fettsäuren erhalten. Eingeschlossen sind auch Mischungen verschiedener polymerer Fettsäuren mit einem überwiegenden Anteil, das heißt mehr als 70 % an dimerer Fettsäure, die zusätzlich einen kleinen Anteil an Monocarbonsäuren aufweisen. Diese Monocarbonsäuren mit 12 bis 22 Kohlenstoffatomen können auch Doppelbindungen sowie Verzweigungen erhalten.

Bei den einzusetzenden Polyetherdiaminen handelt es sich ebenfalls um bekannte Verbindungen. Als typische Vertreter der Polyetherdiamine sind Bis-(2-aminopropyl)-polyoxypropylene und Bis-(3-aminopropyl)-polytetrahydrofurane zu nennen, die ein zwischen etwa 500 und 5 000 liegendes Molekulargewicht haben. Sie sind wegen ihrer leichten Verfügbarkeit bevorzugt. Selbstverständlich sind auch zwei endständige Aminogruppen enthaltende aus polymeren, gegebenenfalls verzweigtkettigen Butandiolen, Pentandiolen und Hexandiolen aufgebaute Polyether geeignet.

Schließlich können bei der Polyamidherstellung neben den Etherdiaminen mit einer linearen oder verzweigten Kette mit mehr als zwei Kohlenstoffatomen, wie zum Beispiel Ethylendiamin, 1,3-Diaminopropan und/oder 1,4-Diaminobutan, Neopentyldiamin, Hexamethylendiamin, Trimethylhexamethylendiamin mitverwendet werden. Weiterhin kommen Diamine in Betracht, die aus dimerisierten Fettsäuren erhalten wurden, wobei die Carboxylgruppen durch Amingruppen substituiert wurden. Produkte dieser Art werden häufig als Dimerdiamine bezeichnet. Als

...

Beispiele für cycloaliphatische Diamine können ferner Diaminodicyclohexylmethan, 3-Aminomethyl-3,5,5-trimethylcyclohexylamin, für aromatische Diamine, Diaminodiphenylmethan, für arylaliphatische Amine Xylylendiamin und für heterocyclische Amine Piperazin, Dimethylaminopiperazin und Dipiperidylpropan genannt werden.

Zusätzlich zu den dimerisierten Fettsäuren können noch aliphatische Dicarbonsäuren mitverwendet werden. Hier kommen in erster Linie in Betracht: Adipinsäure, Azelainsäure, Sebacinsäure, Decandicarbonsäure.

Die erfindungsgemäß zu verwendenden Polyamide werden nach bekannten Verfahren durch Schmelzkondensation hergestellt. Dabei reagieren die Säurekomponenten mit den Aminokomponenten bei Temperaturen von 150 bis 250 °C, wobei das Reaktionswasser durch Destillation oder falls notwendig unter Verwendung eines azeotropen Lösungsmittels und/oder eines Vakuums entfernt werden kann. Durch geeignete Reaktionsführung und die Wahl eines geringfügigen Überschusses von Säure- beziehungsweise Aminfunktion kann in bekannter Weise auf Amin- beziehungsweise Säurezahl Einfluß genommen werden. Die Aminzahl soll Werte zwischen etwa 0,3 und 10, vorzugsweise zwischen 0,5 und 6 aufweisen und die Säurezahl zwischen etwa 0,5 und 15, vorzugsweise zwischen 1,0 und 10 liegen. Der Erweichungspunkt sollte oberhalb von 80 °C liegen und 150 °C nicht übersteigen.

Die erfindungsgemäß einzusetzenden Polyamide zeichnen sich nicht nur durch einen günstigen Erweichungspunkt aus, sondern weisen auch eine gute Verarbeitungsviskosität auf, das heißt sie werden sprunghaft nach dem Überschreiten des Erweichungsintervalls sehr niedrig viskos. Im

...

allgemeinen haben sie ein gutes Filmbildungsvermögen und zeigen eine glatte Oberfläche. Die Adhäsion zu den meisten Substraten ist sehr gut, so daß sie sich auf den verschiedensten Oberflächen verankern, ohne daß eine spezielle Vorbehandlung durchgeführt wird. Auch ist die chemische Beständigkeit sehr gut, das heißt insbesondere eine Beständigkeit gegen Alkali und Säuren.

Die meisten der bekannten Duft- und Riechstoffe lassen sich leicht in einer Konzentration von 1 bis 30 Gewichtsprozent, bezogen auf das Polyamid einarbeiten. In manchen Fällen ist es günstig, zusätzlich noch geringere Mengen bis zu etwa 25 %, bezogen auf das Polyamid an bekannten Weichmachern mitzuverwenden. Dabei können beispielsweise epoxidierte Fettsäureester, die gleichzeitig als Stabilisatoren wirken oder auch Phosphorsäure ester oder Sulfonsäureester von Phenolen verwendet werden. Gewünschtenfalls sind auch Pigmente oder Füllstoffe, wie beispielsweise Kreide oder Bariumsulfat oder auch kieselsäurehaltige Füllstüffe wie gefällte Kieselsäure oder Calciumsilikat, Natrium, Aluminiumsilikat oder auch Neuenburger Kreide mitverwendbar. Gegebenenfalls können auch Farbpigmente wie Eisenoxid oder Phthalocyanine zwecks Farbgebung eingesetzt werden.

Wegen der guten Affinität der meisten Duft- und Riechstoffe zu den Polyamiden sind die Verluste bei der Verarbeitung der Gemische aus Polyamiden, Duft- und Riechstoffen gegebenenfalls weiteren Hilfsstoffen ausgesprochen gering. Dadurch ist die Geruchsbelästigung der mit der Verarbeitung befassten Personen ziemlich gering, verglichen mit den entsprechenden Arbeiten mit anderen Trägersubstanzen auf Polymerbasis.

...

Das Release-Verhalten wurde bei den Gemischen aus Polyamiden und Duft- beziehungsweise Riechstoffen einmal nach der gravimetrischen Methode und zum anderen nach einer subjektiven Methode durch Testpersonen beurteilt. Bei der gravimetrischen Methode werden die Proben gegebenenfalls bei Wärmebelastung für einen längeren Zeitraum beobachtet und die relative Gewichtsabnahme festgestellt. Diese Methode eignet sich insbesondere dann, wenn höhere Mengen an Duft- beziehungsweise Riechstoffen in das Polyamid eingearbeitet worden sind. Insbesondere bei intensiv duftenden Riechstoffen läßt man die Abnahme des Duftempfindens durch Testpersonen beurteilen. Auch diese Methode führt zu guten reproduzierbaren Werten und zeigt die Überlegenheit der erfindungsgemäß zu verwendenden Polyamide als Trägerstoffe.

Für die erfindungsgemäße Verwendung der thermoplastischen Polyamide, die einen Duft- beziehungsweise Riechstoff fixiert enthalten, sind folgende Anwendungen möglich:

- Parfümierung von festen Wasch- und Reinigungsmitteln:

  Durch Auftrag eines duftstoffhaltigen Filmes auf die obere Verpackungswandung oder unter den Verschlußdeckel von Wasch- und Reinigungsmittelverpackungen. Durch die guten adhäsiven Eigenschaften der thermoplastischen Polyamide ist die Haftung auf den verschiedensten Verpackungsmaterialien (saugende und nicht saugende Oberflächen) gegeben.

- Geruchsüberdeckung in Geschirrspülautomaten:

  Durch Einbringung eines duftstofftragenden Filmes auf

...

die Innenwand einer Geschirrspülmaschine. Da die duftenden Polyamide unter den alkalischen Bedingungen der Spülmaschine resistent sind, überdauern sie zahlreiche Spülgänge.

- <u>Wäschebeduftung im Tumbler:</u>
  Durch Einbringen eines mit Duftstoff versehenen Polyamidvlieses oder durch Beschichtung eines rückseitig mit Haftklebstoff beschichteten Bandes beziehungsweise Schaumstreifens.

- <u>Innenbeschichtung von Folienträgern:</u>
  Auf dem inneren Träger einer Papier- oder Folienrolle wird mit Duftstoff versehenes Polyamid aufgetragen.

- <u>Anbringen in Haushaltsmaschinen:</u>
  Nach früher erfolgter Installierung erfolgt ein Auftrag des geschmolzenen Polyamids oder eines mit Kleber versehenen Polyamidstreifens zur nachträglichen Geruchsverbesserung.

- <u>Duftfreigabe im Staubsauger:</u>
  Mit Duftstoff versehenes Granulat wird in den Luftstrom (Beutel) von Staubsaugern gegeben, wodurch bei Gebrauch auf längere Zeit eine Duftnote dem austretenden Luftstrom beigegeben wird.

- <u>Geruchsüberdeckung in chemischen Reinigungsanlagen:</u>
  Nach dem Reinigen mit halogenisierten Kohlenwasserstoffen kann die Trocknung in Gegenwart der dufthaltigen thermoplastischen Polyamide erfolgen. Die Polyamide sind besonders resistent gegen chemische Reinigungsmittel.

...

## Beispiele

Ein für den erfindungsgemäßen Zweck brauchbares Polyamid wurde hergestellt durch Kondensation von

| | | |
|---|---|---|
| 759 | g | dimerisierter Fettsäure (72 % dimere Anteile) |
| 60 | g | Tallölfettsäure sowie |
| 81 | g | Ethylendiamin und |
| 112 | g | Bis-(3-aminopropyl)-polytetrahydrofuran (Molgewicht 750). |

Die Kondensation wurde zunächst bei 60 °C unter Stickstoffatmosphäre begonnen und die Temperatur innerhalb von 1 Stunde auf 230 °C gesteigert und dabei eine Stunde gehalten. Das Reaktionsgefäß wurde auf 15mbar evakuiert und flüchtige Anteile abgetrennt.

Das Polyamid hatte eine Säurezahl von 9,8, eine Aminzahl von 0,7 und einen Erweichungspunkt von +105 °C.

Durch Erwärmen von 80 % des Polyamids mit 5 % Adipinsäuredioctylester und 15 % Parfümöl wurde eine Mischung hergestellt, die anschließend in 5 mm dicke Platten gegossen wurde.

Prüfung der duftstoffhaltigen Polyamide hinsichtlich ihres Duftstoffabgabevermögens

Plattenmuster wurden im Umlufttrockenschrank bei 50 °C gelagert und der Gewichtsverlust gemessen, bezogen auf eingesetzten Duftstoff. Als Parfümöl wurde verwendet ein blumiges, frisch riechendes Öl. Der Gewichtsverlust bei der Lagerung im Umlufttrockenschrank, bezogen auf das eingesetzte Parfümöl, betrug:

| | | |
|---|---|---|
| 1 | Tag | 15 % |
| 3 | Tage | 25 % |
| 7 | Tage | 55 % |
| 14 | Tage | 85 % |

...

Bei offener Lagerung in einem auf 25 °C gehaltenen Raum ergaben sich folgende Werte:

| | | |
|---|---|---|
| 1 | Tag | 8 % |
| 1 | Woche | 25 % |
| 5 | Wochen | 50 % |
| 10 | Wochen | 65 %. |

...

## P a t e n t a n s p r ü c h e

1.) Verwendung von an sich bekannten thermoplastischen Polyamiden auf Basis von dimerisierten Fettsäuren und vorwiegend aliphatischen Diaminen als Träger für Duft- und Riechstoffe.

2.) Verwendung von thermoplastischen Polyamiden gemäß Anspruch 1, dadurch gekennzeichnet, daß die aliphatischen Diamine zu einem erheblichen Teil Polyetherdiamine sind.

3.) Verwendung von thermoplastischen Polyamiden nach Anspruch 1 bis 2, dadurch gekennzeichnet, daß man solche Polyamide einsetzt, die durch Kondensation von

a) 40 bis 48 Mol-% dimere Fettsäuren,
b) 2 bis 10 Mol-% monomere Fettsäuren,
c) 4 bis 25 Mol-% Polyetherdiamine und
d) 25 bis 46 Mol-% aliphatische Diamine

hergestellt worden sind.

4.) Verwendung von thermoplastischen Polyamiden nach Anspruch 1 bis 3, dadurch gekennzeichnet, daß man solche Polyamide einsetzt, bei denen die Polyetherdiamin-Komponente der allgemeinen Formel

$$H_2N - R_1 - O - (R\ O)_x - R_2 - NH_2,$$

in der X eine Zahl zwischen 8 und 80, vornehmlich zwischen 8 und 40 und $R_1$ und $R_2$ gleiche oder ver-

...

schiedene aliphatische und/oder cycloaliphatische Kohlenwasserstoffreste und R ein gegebenenfalls verzweigter aliphatischer Kohlenwasserstoffrest mit 1 bis 6 Kohlenstoffatomen darstellt, entspricht.

...